# EUROPEAN PATENT APPLICATION

(11) **EP 4 647 393 A1**
(43) Date of publication of application: **12.11.2025**
(21) Application number: 25174374.6
(22) Date of filing: 06.05.2025
(51) Int. Cl.: B67D 1/07, A61L 2/18, C02F 1/78, A61L 2/20

(54) **SANITATION DEVICE FOR THE SANITATION OF BEVERAGE DISTRIBUTION SYSTEMS**

(30) Priority: 06.05.2024 CZ 20240182
(71) Applicant: Sanixera s.r.o., 10800 Praha 10, Malesice (CZ)
(72) Inventor: PURS, Jiri, 16000 Praha 6, Bubenec (CZ); SIBRAVA, David, 16900 Praha 6, Brevnov (CZ)
(74) Representative: Kratochvil, Vaclav

(57) **Abstract**

Sanitation device for sanitizing beverage distribution systems containing the main section (16) of the sanitation device with a mains water inlet (1) with a flow meter (3), to which a low-flow valve (4) and/or a tuning valve (5) is connected, behind which is an ozone generator (6) connected to the water outlet (9) to the second section (17) with at least one keg coupler adapter, to which a bubble generator (15) is further connected. The main section (16) of the sanitation device includes a main control unit (8) for enabling communication with a supervisory system, which is a backend (10) suitable for controlling the sanitation process, connected to a low-flow valve (4) by an ozone generator (6) and a bubble generator (15). The second section (17) with keg coupler (19) adapters contains a treated water inlet (11) with ozone connected to the controlled valves (12) of the sanitation outlets (14), wherein the control of the water outlet (9) is connected to an auxiliary control unit (13), which is connected to the main control unit (8), to which the control of the controlled valves (12) is connected.

## Description

### Field of the Invention

The invention relates to sanitation, i.e. cleaning and disinfection, particularly in restaurant and brewery operations, and more specifically to the sanitation of beverage distribution systems.

### Background of the Invention

In the relevant technical field, the following procedures are currently predominantly used.

Chemical sanitation, where the beverage distribution system is filled with a combination of different types of chemicals that subsequently eliminate bacteria present in it; this type of sanitation uses either a special sanitizing container, by means of which the distribution system is filled with sanitizing solution, which then acts there, or uses a pump that repeatedly drives the sanitizing solution through the beverage distribution system.

Mechanical sanitation, which is less frequent, where a sanitation device based on a high-pressure water pump, such as diaphragm, piston, or other type, is used. This pump ensures that this distribution system is cleaned by using a special cleaning sponge, which is pushed through the beverage distribution system by the pump.

Chemical-mechanical sanitation, where a combination of a high-pressure pump and the simultaneous addition of chemicals to the circuit, is used. This type of sanitation is the most efficient, but it is the most time-consuming and logistically demanding.

The mentioned types have several disadvantages.

Safety risks. The use of chemicals poses a risk to end customers. Insufficient flushing after chemical sanitation can lead to contamination of beverages, which poses a health risk to consumers. In some countries, injury cases are so common that breweries allocate part of their budget for compensation.

Economic and logistical disadvantages. The need to add chemicals itself increases logistical costs; chemicals need to be purchased, stored, transported, etc. As handling chemicals poses a safety risk, personnel performing sanitation must be aware of this risk and must be trained to prevent potential accidents. For this reason, external companies are often used to carry out sanitation; however, this entails additional financial and organizational burdens.

Environmental impacts. The use of chemicals poses an environmental burden. If more modern standards regarding ecological disposal are introduced in the future, it is conceivable that ensuring compliance with these standards will bring with it additional complications of a logistical, financial and organizational nature.

Time-consuming. The low level of automation of the sanitation process means a high time requirement. For example, when using a combination of chemical and mechanical sanitation, it is necessary to partially dismantle the beverage distribution system, i.e. at least dismantle the taps. Furthermore, it is typically necessary to repeatedly change the direction of the cleaning solution flow using a manual switch so that the cleaning sponge passes through the beverage distribution system several times. The time-consuming nature of the process is further increased by the need to use protective equipment and the need to record the performed sanitation in the sanitation book.

The issue of so-called beer stone. The action of compounds and mixtures with highly alkaline pH during the sanitation of beer distribution systems can cause the formation of deposits, called Beerstone, inside these distribution systems; these deposits support the resistance of bacterial cultures because they form structures on the walls of the distribution lines in which bacteria can often survive despite standard sanitation. Therefore, it is recommended to alternate agents with acidic and alkaline pH, which further increases the demands on logistics and personnel.

Problems of using sanitizing sponges. For perfect cleaning of beverage distribution systems, it is advisable to use combined sanitation, i.e. sanitizing sponges plus chemicals; however, in extreme cases, the sponges can get stuck in the pipes and thus force their disassembly. The use of sponges further increases the time required for the sanitation process; for example, it is necessary to disassemble the foam on beer (FOBs) detectors, if present, and wash them separately before sanitizing with sponges.

Absence of reliable control mechanisms. The only mechanism for checking the correct execution of sanitation is often operator statements or paper records, i.e. sanitation books and protocols, which have a lower informative value regarding the correct and timely execution of sanitation.

### Summary of the Invention

The aforementioned shortcomings are largely corrected by the sanitation device for sanitizing beverage distribution systems, according to the present invention. Its essence is that it contains the main section of the sanitation device with the mains water inlet with a flow meter, to which a low-flow valve and a tuning valve are connected, behind which there is an ozone generator connected to the water outlet to the second section with keg coupler adapters, to which a bubble generator, such as microbubbles and nanobubbles, is further connected. The main section of the sanitation device also contains a main control unit equipped with a control panel and connected to a low-flow valve by the ozone generator and a bubble generator. The second section with keg coupler adapters contains a treated water inlet connected to the controlled valves of the sanitation outlets, the water outlet being connected to an auxiliary control unit, connected to the main control unit. The control of controlled valves is connected to the auxiliary control unit.

In one advantageous embodiment, the main control unit can also communicate with the supervisory system, which is a backend.

A high-flow valve is advantageously located between the flow meter and the bubble generator, connected to the main control unit.

In another embodiment, a closed sanitation circuit comprising a container for collecting sanitizing fluid is connected to the bubble generator via a pump in an advantageous embodiment, to which the sanitizing fluid inlet and the auxiliary flow meter are connected, the container being provided with a drain valve connected to the main control unit.

The keg couplers are, in one particularly advantageous embodiment, equipped with a communication device with an IR identifier of the keg couplers.

The presented sanitation system allows automatic or semi-automatic sanitation of beverage distribution systems using a combination of dissolved ozone water cycles and different types of bubbles. In one particularly advantageous embodiment, the bubble generator is capable of generating different bubble sizes, including microbubbles and nanobubbles (ultra-fine-bubbles, UFBs). The combination of cycles and the time duration of each cycle creates a process which, by its mechanical and chemical properties, sanitizes, i.e. disinfects and cleans, the beverage distribution system and removes sediments, deposits, i.e. plaques, from them by their gradual disruption until their complete removal, which is defined by the relevant hygiene standards. The combination, sequencing and timing of the cycles is controlled by the main control unit.

Beverage distribution refers to an assembly of pipes, hoses, couplings and other parts from an adapter connected to the container, storing the beverage, which can be, such as a keg coupler for keg tapping, to end devices, for example beverage dispensing taps. Thus, the beverage distribution system includes all distribution parts that come into contact with dispensed beverages during normal operation, including any components of cooling equipment. The presented sanitation system can perform the sanitation automatically or in semi-automatic mode with only the minimum number of manual steps necessary to start the sanitation and without the need for further manual intervention during the sanitation process.

The main control unit can perform the appropriate steps necessary for sanitization, check their correct execution, and can autonomously generate a report of the performed sanitation and send it to a remote server or cloud storage, i.e. a supervisory control system, which is a backend, using data transfer, e-mail message, etc.

For communication, the main control unit uses WiFi/LTE technology in one particularly advantageous embodiment. However, it is clear to an expert in the field that it could also use other technologies, such as LTE CAT M, Nb-lot or others. If connectivity is lost, the main control unit collects the data in one particularly advantageous embodiment into the internal memory and then sends the data to the supervisory system when the connection is restored. All communication with the supervisory system, which is a backend, is encrypted.

The sanitation device includes a display and buttons that make up the physical user interface of the device. This can be used to start or interrupt sanitation and to display basic status information. Optionally, the sanitation device also allows connection of wired or wireless sensors such as thermometers, mechanical flow meters and more. Information about the performed sanitation and sensor data can optionally be stored in a database that is part of the backend. In this case, the collected data can be further displayed and analyzed. Optionally, the device allows remote initiation of sanitation, for example via a mobile phone.

The sanitation device allows setting advanced sanitation parameters that can be specific to each establishment, e.g. restaurant, via a web interface with limited access or a mobile application. This prevents unintended modification of these parameters while allowing their remote configuration.

Sanitation device is structurally designed in the standard, ensuring its resistance to water ingress (IP67).

Sanitation device is modular - it consists of a main section and a second section with sanitation adapters. This allows it to support different types and numbers of keg couplers.

The main section of the sanitation device cleans the beverage distribution system using a combination of an ozone generator and a specialized bubble generator, which in one possible embodiment provides the formation of microbubbles and nanobubbles, among others. An ozone generator creates ozone from regular water, i.e. from a standard mains water supply, while simultaneously dissolving the ozone in the fluid. In one particularly advantageous embodiment, the ozone generator is of the electrolytic type. This electrolytic type ozone generator can safely generate high levels of ozone - up to 300 mg/h. The flow rate depends on the type of generator used, in one case the implementation is 60-100 liters per hour. The water thus obtained with dissolved ozone is used for disinfection of beverage distribution systems. Sanitation device can also independently generate bubbles in the water, including microbubbles and nanobubbles, which , through a combination of mechanical action and other effects increase the efficiency of the sanitation process. Automation also allows repeating sanitation cycles, consisting of filling the beverage distribution system with ozone water and then flushing it with water with different types of bubbles. As part of the automated treatment process, it is also possible to collect wastewater with ozone after sanitation. The water can be stored for as long as necessary to convert ozone to oxygen spontaneously and then automatically discharged in accordance with applicable standards.

In one implementation variant, the automated sanitation system also includes a pump. This pump allows a significant increase in sanitation efficiency without a disproportionate increase in water consumption or sanitation time. For example, the pump can be of the diaphragm type, with a diaphragm made of Santoprene; this material has a very high resistance to ozone.

In an alternative implementation, a pump such as a centrifugal type may be used; some pumps of this type generate microbubbles which also have a cleaning effect.

In an alternative implementation, the ozone generator can be connected in a configuration where water is repeatedly driven through it by a pump in the circuit. This configuration may bring advantages in the form of greater sanitation efficiency, but may not be suitable for all types of ozone generators, as in some cases it will lead to disproportionate wear and tear.

Sanitation device according to this technical solution allows safe, automatic or semi-automatic sanitation of beverage distribution systems using a combination of cycles of water with dissolved ozone, water with bubbles of various types, including microbubbles and nanobubbles. The sequence of the cycles and their duration creates a combination which, with its mechanical and chemical properties, sanitizes the beverage distribution system, i.e. disinfects and cleans and removes sediments, deposits, i.e. plaques, biofilm from them by gradually disrupting them layer by layer until they are removed. The combination, sequencing and timing of cycles is managed by the control unit. Ozone is generated directly in the device - ozone generator. Bubbles are created by a bubble generator, which is also part of the device. Bubbles are directly injected into the cleaning fluid, i.e. ozone-carbonated water, and contribute to the removal of sediments, plaque and biofilm. Disposal is defined by meeting the hygiene standards applicable to the location and country.

The effect can be programmed as needed by setting several cycles, each of which involves filling the beverage distribution systems with ozone water and the subsequent mechanical action of different types of bubbles for a set period of time. Fluid used for sanitation may be collected in a container, which may or may not be part of the device, until the dissolved ozone spontaneously converts to oxygen. Subsequently, this fluid is automatically discharged into the waste. All processes and cycles of the central unit are adjustable and programmable in the supervisory system, which is a backend. After the sanitation process is completed, a report of the performed sanitation is automatically generated and then sent to the appropriate supervisory system. The device communicates with the supervisory system, which is a backend using LTE, GSM and WiFi, and thus allows sending reports of the performed sanitation. Communication is encrypted. The device is adapted for operation in IP67 category.

The advantage of one implementation of the solution is also the possibility of collecting data from sensors and their analysis, where the technology of automatic detection of keg couplers is used, optionally with an extension of their identification system.

The use of an electrolytic ozone generator ensures high system safety and completely prevents the accidental formation of hazardous concentrations of ozone gas.

### Brief Description of the Drawings

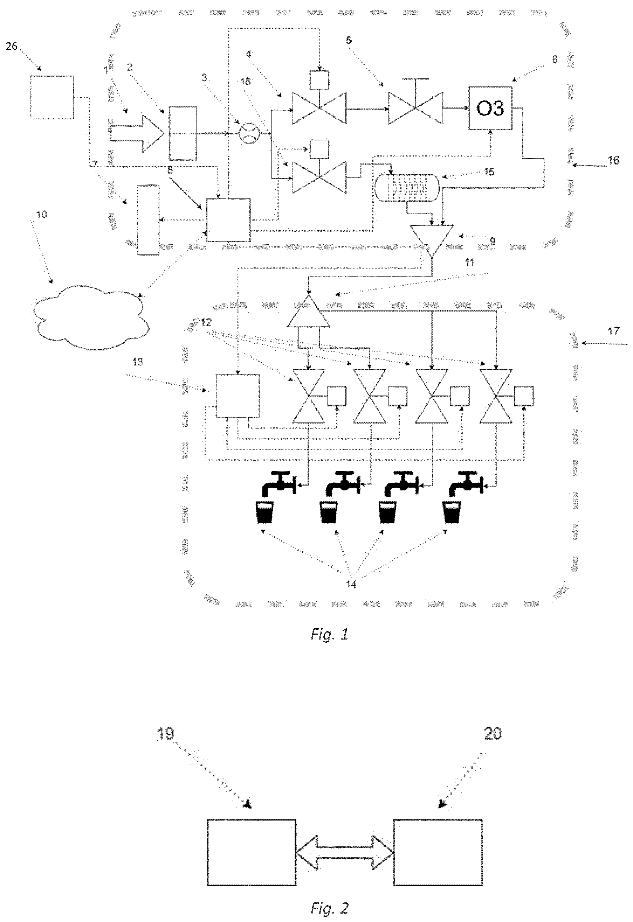

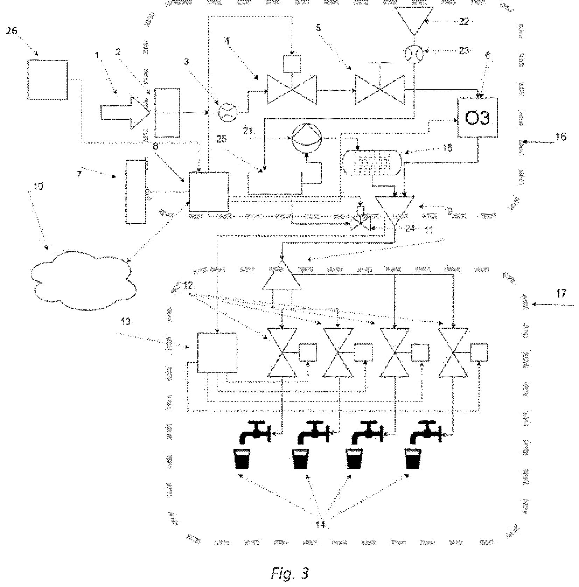

Fig. 1 shows a diagram of a sanitation device without an auxiliary pump. Fig. 2 shows a diagram of the communication between the keg coupler and the sanitation device and Fig. 3 shows a diagram of a sanitation device with an auxiliary pump.

### Exemplary Embodiments of the Invention

An exemplary sanitation device for sanitizing beverage distribution systems includes a main section 16 of a sanitation device with a mains water inlet 1 with a flow meter 3, to which a low-flow valve 4 and a tuning valve 5 are connected, behind which an ozone generator 6 is connected to a water outlet 9 to the second section 17 with keg coupler adapters, to which a microbubble and nanobubble generator 15 is further connected. The main section 16 of the sanitation device also contains the main control unit 8 with a supervisory system, which is a backend 10 equipped with a control panel 7 and connected to the low-flow valve 4 by the ozone generator 6 and the microbubble and nanobubble generator 15. The second section 17 with keg coupler 19 adapters contains a treated water inlet 11 connected to the controlled valves 12 of the sanitation outlets 14, the water outlet 9 being connected to an auxiliary control unit 13, connected to the main control unit 8. The control of the controlled valves 12 is connected to the auxiliary control unit 13. A high-flow valve 18 is located between the flow meter 3 and the bubble generator 15, connected to the main control unit8.

In another embodiment, instead of the high-pressure valve 18, a closed sanitation circuit containing a container 25 for collecting sanitizing fluid is connected to the microbubble and nanobubble generator 15 via the pump 21, to which the sanitizing fluid inlet 22 with the auxiliary flow meter 23 is connected. The container 25 is provided with a drain valve 24 connected to the main control unit 8.

The keg couplers 19 are provided with a communication device with an IR identifier 20 of the keg couplers 19.

In both embodiments, the devices are powered by a waterproof power supply 26, which provides power to the devices while ensuring electrical safety.

The mains water inlet 1 allows the connection of pressurized water for sanitation. Inlet filter 2 filters microscopic particles and reduces water hardness, thereby increasing the lifetime of the ozone generator 6. The flow meter 3 measures the flow velocity of the fluid, allows detection of the correct connection of the keg couplers 19 and can operate on any principle, for example, an ultrasonic flow meter, described in Utility Model No. 37258. Low-flow valve 4 for low flow, together with high-flow valve 18 for high flow, allow switching between two flow rates - for sanitation and subsequent flushing. The flow rate tuning valve 5 allows manual fine-tuning of the flow rate in the case of an open low-flow valve 4 on site, as the flow rate directly affects the concentration of generated ozone. The electrolytic ozone generator 6 generates ozone and dissolves it into water. The physical user interface with a control panel 7 displays status information and allows sanitation to start and stop. The main control unit 8 controls the sanitation process, switches valves 4, 18, arranges the operation of the physical user interface, arranges the communication of the sanitation device with the backend 10 and transmits commands to the auxiliary control unit 13. The water outlet 9 is used to transfer the water from the main section 16 to the second section 17 with keg coupler adapters. The supervisory system, which is a backend 10, provides communication between the mobile application and the sanitation device, allowing the reception and storage of sanitation protocols, the collection of sensor data and their storage, and the remote control of the sanitation device. Backend 10 itself is an extension of the backend described in Utility Model No. 37258. The treated water inlet 11 is supplied to the section with keg coupler 19 adapters. Controlled valves 12 of the second section 17 with keg coupler 19 adapters allow switching which tap water flows into. The auxiliary control unit 13 of the second section 17 with keg coupler 19 adapters is controlled by the main control unit 8 and controls the switching of the individual controlled valves 12. Sanitation outlets 14 with optional identifiers 20 of the keg couplers 19 allow the connection of the keg couplers 19 for the sanitation, optionally also allow the identification of the smart keg couplers 19. The microbubble and nanobubble ozone generator 6 with optional particulate filter 2 generates nanobubbles from the surrounding air to help remove the biofilm inside the beer distribution system. Ozone generator 6 can be based on Venturi, cavitation or other principle. It is particularly advantageous to use a type of generator into which it is possible to inject gas under pressure and then change the size of the generated bubbles by changing the pressure. The number of nanobubbles generated by a UFB generator is in he order of hundreds of millions to billions per milliliter. It may include a particulate filter, which reduces the amount of foreign particles that enter the beverage distribution system when air is sucked in. The ozone generator 6 may optionally include a compressor with power controlled by the main control unit 8. This allows the main control unit 8 to automatically modulate the ratio of the number of generated nanobubbles, microbubbles and standard-sized bubbles. The IR identifier 20 of the smart keg coupler 19 can detect and identify the smart keg coupler 19, which transmits an IR code that differs between the individual keg couplers 19 within the restaurant establishment. Through it, the IR identifier 20 can distinguish the individual keg coupler 19 from each other. The keg coupler 19, for example, by means of a standard IR led, or more preferably a larger number of such diodes, for example at a wavelength of 940 nm, periodically transmits a sequence where the diode or diodes are repeatedly switched on and off. The specific form of this sequence, i.e. the length of the sections along which the diode is turned on or off and any other parameters, then encodes data that uniquely indicate any keg coupler. In one possible implementation, the diodes are controlled by a microcontroller, where this unit, i.e. the diode + microcontroller and any other supporting circuits, is powered by a battery.

The transmitted sequence is received by the identifier 20 of the keg coupler by a receiving element, which in one particular implementation may be, for example, an infrared photodiode, a phototransistor, or another specialized receiving element with a built-in demodulator.

The identifier 20 of the keg coupler transmits the read information to the main unit 8.

In the second embodiment, the pump 21 pumps sanitizing fluid, i.e. water with ozone or various types of bubbles, through a closed circuit beverage line. Pump 21 can be of different types, especially suitable is the use of a high-pressure diaphragm pump with a diaphragm made of ozone-resistant material, e.g. Santoprene. The pictured location of the pump 21 in the system is only one of the possible ones; alternatively, it can be connected to drive the sanitizing fluid in the opposite direction to the regular beverage flow, which in some cases can further increase sanitation efficiency.

The sanitation circuit is provided with an inlet 22 for closing, which allows the connection of a hose that connects the end of the beverage line back to the container 25 for collecting sanitizing fluid. Auxiliary flow meter 23 allows detection of possible circuit disconnection. The container 25 is provided with a drain valve 24 for draining the container 25, which serves for collecting the sanitizing fluid and enabling the closure of the sanitizing circuit and ensuring the collection of the fluid with ozone for the time necessary for its spontaneous conversion to oxygen.

The following text is a more detailed description of the functionality of individual parts of the system.

Overview of the function of the whole system and its use

The restaurant has a sanitation device. The device is optionally connected to the Internet via LTE/WiFi technologies and communicates with the supervisory system, which is a backend 10. Sensors, such as thermometers, mechanical flow meters and others, are optionally connected to the device using wired and/or wireless technology, such as BLE. The sanitation device can serve as an intermediary for sending data from these sensors to the supervisory system, which is a backend 10. The sanitation device can be used at any time to sanitize beverage distribution systems. The supervisory system, which is a backend 10 in cooperation with sanitation devices and a mobile application, can optionally alert operators to the impending need for sanitation.

The duration of the sanitation depends on the specific parameters of the restaurant equipment, such as the material of the pipes used, their length, the frequency of sanitization and more. These parameters can be set by authorized persons, for example, using a mobile application. Optionally, the device can support multiple different sanitation modes of varying intensity, which can then be selected via the physical user interface in the form of control panel 7 or a mobile application.

Thanks to the special design of the sanitation outlets 14, it is not necessary to switch off the propellant supply before starting or switch it back on after completion.

Sanitation has several steps, most of which are fully autonomous. These steps vary depending on the variant of the automatic sanitation device, mainly depending on whether the variant includes an auxiliary pump 21 or not.

Option without auxiliary pump
1) The user disconnects the keg couplers 19, connected to the beverage distribution system they wish to sanitize, from the kegs to which they were connected. Subsequently, they connect these keg couplers 19 to the sanitation outlets 14. The user starts sanitization using the physical user interface by pressing the button that is part of it, or optionally using the mobile application. If the restaurant uses foam detectors (FOB), it is necessary to switch them to cleaning mode according to their respective instructions for use. The automatic sanitation device starts the process of detecting open taps. Subsequently, the user may open the taps connected to the couplers that have just been connected to the sanitation outlets.
2) detection of engaged taps
   The sanitation device opens the electric low-flow valve 4 for low flow and starts opening the individual controlled valves 12 connected to the sanitation outlets 14. If the tap is connected to the appropriate sanitation outlet and is open, water will start flowing out. This fact is detected by flow meter 3. Using this procedure, the sanitation device determines which taps are connected and opened and then transmits this information to the operator via the physical user interface and optionally also the mobile application. At this point, the operator can detect any problems, such as improper engagement of the keg coupler 19, correct any problems that may have risen, and perform the detection again. In case of further non-cooperation from the operator, the process will automatically move to the next step after a while. If the keg couplers 19 are "smart", the individual keg couplers 19 are simultaneously identified by means of communication between the smart keg coupler and the identifier 20 of the keg couplers 19, allowing the sanitation device to know which keg coupler 19 is engaged to which sanitation outlet, or which parts of the beer distribution system were cleaned during sanitation. This information will be transmitted at the end of the sanitation to the supervisory system, which is a backend 10 within the sanitation protocol.
3) flushing of beer pipes using UFB technology
   First, all valves 12, 18, 4 are closed. Subsequently, the electric high-flow valve 18 for high flow is opened. The water starts flowing through the UFB generator 15 and then through the outlet 9 from the main unit to the inlet 11 of the unit with keg coupler adapters and from there through the valves 12 of the unit with keg coupler adapters and to the sanitation outlets 14. In this step, the electric high-flow valve 18 for high flow is controlled by the main control unit 8, and the valves 12 of the unit with keg coupler adapters 19 are controlled by the main control unit 8 via the auxiliary control unit 13 of the section with keg coupler 19 adapters. This step fills the beverage distribution systems with ultra-high amounts of nanobubbles, in the order of tens to hundreds of millions of nanobubbles per 1 ml of water. This is followed by flushing the pipeline with high flow rates, typically 300 I/h or more. This step leads to the disruption and partial removal of biofilm and other deposits, which in the next steps will allow for better action of the ozone, as well as the removal of beverage residues from the distribution system, which in the next steps will improve the use of the generated ozone. In this step, all parts of the beverage distribution system are gradually filled with water with nanobubbles, this is ensured by gradually opening the controlled valves 12 of the section with keg coupler 19 adapters through the coordination of the main control unit 8 with the auxiliary control unit 13 of the section with keg coupler 19 adapters while opening the high-flow valve 18 for fast flow through the main control unit8.
4) filling the beverage distribution system with water with dissolved ozone
   electrolytic ozone generator 6 is then automatically activated by control unit 8 and starts generating ozone which dissolves in water. The flow rate is reduced to achieve a sufficiently high concentration of ozone. Flow reduction is ensured by switching to low-flow valve 4 for low flow, along with the proper adjustment of the flow rate tuning valve 5. In this step, all parts of the beverage distribution system are gradually filled with water with dissolved ozone. This is ensured by gradually opening the controlled valves 12 of the section with keg coupler 19 adapters through the coordination of the main control unit 8 with the auxiliary control unit 13 of the section with keg coupler 19 adapters while opening the low-flow valve 4 for slow flow through the main control unit8.
5) reaction of dissolved ozone with biofilm in beverage distribution systems
   Subsequently, a countdown is started; for a predetermined period of time, which depends on the settings, adjustable via the mobile application, the dissolved ozone reacts with the biofilm in the beverage distribution system. The countdown is managed by the main control unit8.
6) removal of decomposed biofilm by UFB technology
   subsequently, the flow rate is again increased by opening the electrically controlled high-flow valve 18 for high flow, and using the bubble generator 15, the beverage distribution systems are filled with bubbles. These bubbles have an abrasive effect and contribute to the removal of the decomposed biofilm. Optionally, a combined bubble generator 15 can be used to create nanobubbles, microbubbles and/or standard-sized bubbles. In addition to nanobubbles with antibacterial properties, this type of bubble generator 15 can also generate large bubbles with high abrasive ability that can remove mechanical impurities very efficiently. In this step, all the beverage distribution systems are gradually filled with water with nanobubbles; this is ensured by gradually opening the controlled valves 12 of the section with keg coupler 19 adapters by means of the interaction of the main control unit 8 with the auxiliary control unit 13 of the section with keg coupler 19 adapters, while simultaneously opening the high-flow valve 18 for fast flow through the main control unit8.
7) disinfection + flushing
   The UFB generation step with some possible variants of the bubble generator 15 uses the intake of surrounding air. Optionally, this bubble generator 15 can be expanded with a microscopic particle filter to prevent bacteria from being sucked in. If this option is disadvantageous, it is possible to alternatively include in the sanitation process an additional step consisting of generating a small amount of ozone by an ozone generator 6 and filling the beverage distribution system with water with this ozone.
   In this step, all parts of the beverage distribution system are gradually filled with water with dissolved ozone using the same procedure as in step 4. The ozone generator 6 is switched off during the flushing process, and the main control unit 8 increases the water flow rate by opening the electrically controlled high-flow valve 18, and, using of the bubble generator 15, the pipe is filled with water with bubbles. At the end of this step, the pipe is filled with clean, ozone-free water, but with nanobubbles. The procedure takes advantage of the phenomenon of high lifetime of nanobubbles and their spontaneous movement in water. Water with nanobubbles has a certain sanitizing effect in itself, so sanitization only proceeds through the interaction of stagnant water with nanobubbles with the remaining impurities on the inner surface of the beverage distribution system.
8) report on performed sanitation
   A report of the performed sanitation is sent to backend 10. If this is not currently possible, for example due to a connectivity failure, the information will be provided additionally.

### Variant with an auxiliary pump 21

The sanitation procedure differs slightly for the variant with the auxiliary pump 21. Auxiliary pump 21 allows for increased sanitation efficiency by longer exposure to water with ozone and nanobubbles, ensuring a higher concentration of nanobubbles and ensuring a high flow rate. This variant of the sanitation device also includes a container 25, which can be advantageously used to collect water with ozone until the ozone concentration falls below a certain level. This can be particularly important if the laws on the location where the sanitation device is deployed limit the maximum concentration of ozone in wastewater.
1) The user disconnects the keg couplers 19, connected to the beverage distribution system they wish to sanitize, from the kegs to which they were connected. Subsequently, they connect these keg couplers 19 to the sanitation outlets 14. The user connects a hose to the taps, leading to the inlet for closing the sanitation circuit. The user opens the taps, connected to the couplers engaged to the sanitation outlets 14. The user starts sanitization using the physical user interface by pressing the button that is part of it, or optionally using the mobile application. The automatic sanitation device starts the process of detecting open taps.
2) detection of engaged taps
   The sanitation device opens the electric low-flow valve 4 for low flow and starts opening the individual valves 12, leading to the sanitation outlets 14. If the tap is connected to the corresponding sanitation outlet 14 and is open, water starts to flow out; this fact is detected by the flow meter 3. The proper closure of the circuit by the user is then verified by the secondary auxiliary flow meter 23. Using this procedure, the sanitation device determines which taps are connected and opened; it then transmits this information to the operator via the physical user interface and optionally also the mobile application. At this point, the operator can detect any problems, such as improper engagement of the keg coupler 19, or incorrect closing of the circuit back to the circuit closure inlet, and can correct any problem and perform the detection again. In case of further non-cooperation from the operator, the process will automatically move to the next step after a while. In the case where the keg couplers 19 are "smart", the individual keg couplers 19 are simultaneously identified by means of communication between the smart keg coupler 19 and the identifier 20 of the keg couplers 19, enabling the sanitation device to know which keg coupler 19 is engaged to which sanitizing outlet 14, and hence which parts of the beverage distribution system were cleaned during sanitization. This information will be transmitted at the end of the sanitation to the supervisory system, which is a backend 10 within the sanitation protocol.
3) flushing of beverage residues from the beverage distribution system
   Subsequently, the main control unit 8 opens the low-flow valve 4 and, with the ozone generator 6 switched off, flushes the beverage distribution lines with water. This ensures that there is not a large amount of beverage in the beverage distribution system that unnecessarily dilutes the water with ozone and thus reduces its efficiency. The water flows back through the beverage distribution system via the circuit closure inlet via the secondary auxiliary flow meter 23 to the container 25. Subsequently, this container 25 is drained by opening the drain valve 24.
4) filling the beverage distribution system with water with dissolved ozone
   electrolytic ozone generator 6 is then automatically activated by control unit 8 and starts generating ozone which dissolves in water. While opening the low-flow valve 4 at the same time in this step, all parts of the beverage distribution system are gradually filled with water with dissolved ozone, this is ensured by a procedure identical to that given in point 4) of the description of the variant without the auxiliary pump 21. Water with ozone flows through the beverage distribution system, through the circuit closure inlet 22, through the secondary auxiliary flow meter 23 to the container 25. The secondary auxiliary flow meter 23 allows the automated sanitation device to detect and respond to any circuit breakage, e.g. by interrupting the sanitization and restoring the system to a safe state.
5) removal of decomposed biofilm by combining UFB + water with dissolved ozone
   Main unit 8 shuts off the low-flow valve 4 and activates the pump 21. This begins to push the dissolved ozone water from the container 25 through the bubble generator 15, beverage distribution system, through circuit closure inlet 22 and secondary auxiliary flow meter 23 back into container 25. The secondary auxiliary flow meter 23 again allows the automated sanitation device to detect and respond to any circuit breakage. Closing the circuit allows for long-term action of water with ozone and nanobubbles without excessive water consumption - water is pumped into the circuit repeatedly. At the same time, more nanobubbles are constantly generated in the water. This allows extremely high concentrations of nanobubbles to be achieved in this water, further increasing the efficiency of the sanitation process. Optionally, it is possible to advantageously use a variant of a bubble generator 15 that can generate a combination of nanobubbles, microbubbles and standard bubbles using a compressor. To increase the efficiency of the process, steps 4-5 can be performed repeatedly until the maximum capacity of the container 25 is filled.
6) disinfection + flushing
   this step aims to fill the beverage distribution system with water with nanobubbles, where these can continue to act until the keg couplers are disconnected from the sanitation device and the beverage is tapped. It also aims to remove water with ozone from the beverage distribution system and to flush out any impurities introduced into the system as a by-product of air intake by the nanobubble generator 15.
   The form of the step is identical to step 7) of the variant without an additional pump; this step is described above.
7) Removal of ozone from the cleaning fluid
   In some countries, limits may be imposed on the concentration of ozone in wastewater. For this purpose, the device may wait for the time required for the ozone concentration to naturally decrease to the permitted level and then drain the container 25 by opening the drain valve 24.
8) sending the report on performed sanitation
   A report of the performed sanitation is sent to backend 10. If this is not currently possible, for example due to a connectivity failure, the information will be provided additionally.

### Industrial Applicability

The device according to the present invention will find application especially in cleaning of restaurant equipment, especially taproom and cooling equipment.

### Reference Signs List

- 1 -: mains water inlet
- 2 -: inlet filter
- 3 -: flow meter
- 4 -: low-flow valve
- 5 -: tuning valve
- 6 -: ozone generator
- 7 -: control panel
- 8 -: main control unit
- 9 -: water outlet to the section with keg coupler adapters
- 10 -: supervisory system, which is a backend
- 11 -: treated water inlet
- 12 -: controlled valves
- 13 -: auxiliary control unit
- 14 -: sanitation outlets
- 15 -: bubble generator
- 16 -: main section of the sanitation device
- 17 -: second section with keg coupler adapters
- 18 -: high-flow valve
- 19 -: smart keg coupler
- 20 -: IR identifier of the smart keg coupler
- 21 -: pump
- 22 -: inlet of the closed sanitation circuit
- 23 -: auxiliary flow meter
- 24 -: drain valve
- 25 -: container for collecting sanitizing fluid
- 26 -: power supply

## Claims

1. A sanitation device for sanitizing beverage distribution systems comprising
- a main section (16) of the sanitation device with a mains water inlet (1) with a flow meter (3),
- a low-flow valve (4),
- a tuning valve (5); and
- a main control unit (8),
**characterised in that** the low-flow valve (4) is connected to a flow meter (3), and the low-flow valve (4) is connected to a tuning valve (5), behind which is an ozone generator (6) connected to a water outlet (9) to a second section (17) of the sanitation device with at least one keg coupler adapter, where the water outlet (9) is further connected to a bubble generator (15), while the main section (16) of the sanitation device contains the main control unit (8), which is adapted for communication with a supervisory system - backend (10) for monitoring the sanitation process and is connected to the low-flow valve (4), to the ozone generator (6), and to the bubble generator (15), while the second section (17) with keg coupler adapters contains a treated water inlet (11) with ozone connected to the controlled valves (12) of sanitation outlets (14), while the control of the water outlet (9) is connected to an auxiliary control unit (13), which is connected to the main control unit (8), and the auxiliary control unit (13) is further connected to a control of controlled valves (12).

2. The sanitation device according to claim 1, **characterized in that** it includes a control panel (7).

3. The sanitation device according to any one of claims 1 or 2, **characterized in that** a high-flow valve (18) connected to the main control unit (8) is located between the flow meter (3) and the bubble generator (15).

4. The sanitation device according to claim 1, **characterized in that** the bubble generator (15) is connected via a pump (21) to a closed sanitizing circuit containing a container (25) for collecting a sanitizing fluid, a sanitizing fluid inlet (22) with an auxiliary flow meter (23) is connected to the container (25), while the container (25) is provided with a drain valve (24) connected to the main control unit (8).

5. The sanitation device according to any one of claims 1 to 4, **characterized in that** the bubble generator (15) generates bubbles, microbubbles and/or nanobubbles.
